(19) Europäisches Patentamt
European Patent Office
Office européen des brevets

(11) EP 4 502 022 A1

(12) **EUROPEAN PATENT APPLICATION**
published in accordance with Art. 153(4) EPC

(43) Date of publication:
**05.02.2025 Bulletin 2025/06**

(21) Application number: **22935537.5**

(22) Date of filing: **31.03.2022**

(51) International Patent Classification (IPC):
***C08J 9/00*** *(2006.01)*

(52) Cooperative Patent Classification (CPC):
**C08J 9/00**

(86) International application number:
**PCT/JP2022/016907**

(87) International publication number:
**WO 2023/188399 (05.10.2023 Gazette 2023/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kao Corporation**
**Tokyo 103-8210 (JP)**

(72) Inventors:
- **MURAKAWA, Kohei**
**Haga-gun, Tochigi 321-3497 (JP)**
- **NIITSU, Taichi**
**Haga-gun, Tochigi 321-3497 (JP)**
- **TAKADA, Yuki**
**Haga-gun, Tochigi 321-3497 (JP)**

(74) Representative: **Vossius & Partner Patentanwälte Rechtsanwälte mbB**
**Siebertstrasse 3**
**81675 München (DE)**

# (54) POROUS FILM

(57) A porous film includes at least an olefin-based resin composition, an inorganic filler, and a triglyceride. With respect to 100 parts by mass of the olefin-based resin composition, the content of the inorganic filler is from 50 to 400 parts by mass and the content of the triglyceride is from 0.1 to 30 parts by mass. The triglyceride includes a group derived from a fatty acid having from 16 to 22 carbon atoms, the group being a hydrocarbon group having neither an unsaturated bond nor a substituent. The porous film is suitable for use as a constituent material of an absorbent article.

EP 4 502 022 A1

## Description

### Technical Field

The present invention relates to a porous film.

### Background Art

Porous films having high water repellency and moisture permeability are known in the art. Such porous films are typically manufactured by subjecting a resin film containing a filler and an additive to uniaxial or biaxial stretching, to form a multitude of micropores therein.

For example, Patent Literature 1 discloses a method of manufacturing a porous film by obtaining a film by melting and shaping a composition containing a polyolefin resin, a filler, and a triglyceride, and stretching the obtained film into a sheet-like form. In this Literature, the triglyceride is blended with the aim of uniformly dispersing the filler within the resin and the aim of improving anisotropy of the porous film.

Patent Literature 2 discloses a porous film obtained by forming a film by melting and shaping a composition containing a polyolefin resin, a filler, and a third component, and then stretching the film. The third component is a mixture of (A) hydrogenated castor oil, and (B) an ester of a carboxylic acid having 8 or more carbon atoms and including no hydroxy group and a polyol having 3 or fewer carbon atoms. In this Literature, the third component is used with the aim of solving the problem that the porous film becomes transparent.

Patent Literature 3 discloses a porous film made from a resin composition containing polyethylene, an inorganic filler, and a plasticizer, wherein hydrogenated castor oil, including a triglyceride of glycerin and 12-hydroxyoctadecanoic acid as a main component, is used as the plasticizer. In this Literature, the hydrogenated castor oil is used with the aim of improving the plasticity and ductility/malleability of the porous film.

### Citation List

#### Patent Literature

Patent Literature 1: JP S62-10141A

Patent Literature 2: JP 2001-302828A

Patent Literature 3: JP 2016-089009A

### Summary of Invention

The present invention provides a porous film including at least an olefin-based resin composition, an inorganic filler, and a triglyceride, wherein:

a content of the filler is from 50 to 400 parts by mass with respect to 100 parts by mass of the olefin-based resin composition;
a content of the triglyceride is from 0.1 to 30 parts by mass with respect to 100 parts by mass of the olefin-based resin composition; and
the triglyceride includes a group derived from a fatty acid having from 16 to 22 carbon atoms, the group being a hydrocarbon group having neither an unsaturated bond nor a substituent..

The present invention also provides an absorbent article including the aforementioned porous film.

### Description of Embodiments

As disclosed in Patent Literatures 1 to 3 described above, techniques for including a triglyceride in a porous film with the aim of improving properties, such as the porous film's ductility/malleability, are known in the art. There is, however, a need for a porous film having better leakage preventability than ever before while maintaining satisfactory moisture permeability.

The present invention therefore relates to an improvement in porous films containing a triglyceride, and more

specifically, relates to a porous film having better leakage preventability than ever before while maintaining satisfactory moisture permeability.

The present invention is described below according to preferred embodiments thereof. A porous film of the present invention has a multitude of micropores. These micropores provide the porous film of the present invention with moisture permeability. The porous film of the present invention also has excellent water repellency and excellent leakage preventability. The porous film of the present invention includes at least an olefin-based resin composition, an inorganic filler, and a triglyceride. In addition to these components, the porous film of the present invention may include various additives with the aim of improving various properties of the porous film.

In the specification of the present application, "olefin-based resin composition" encompasses both cases including only a single type selected from various olefin-based resins and cases including two or more types thereof. "Olefin-based resin composition" is a concept that includes only one or more of various olefin-based resins and does not include other resins and components other than resin. Note, however, that the porous film of the present invention is not prohibited from including resins other than the olefin-based resin.

The olefin-based resin composition to be used in the present invention includes, as a main component, a polymer or a copolymer of a monoolefin such as ethylene, propylene, butene, etc., and examples may include high-density polyethylene, low-density polyethylene, linear low-density polyethylene, polypropylene, and mixtures discretionarily including two or more types of the above in combination.

From the viewpoint of being able to impart satisfactory flexibility to the porous film of the present invention and from the viewpoint of improving bleed-out properties of triglyceride (described below) and improving water repellency of the porous film, it is preferable that the olefin-based resin composition to be used in the present invention has a low density. From this viewpoint, it is preferable that the density of the olefin-based resin composition is preferably less than 0.900 g/cm$^3$, more preferably 0.895 g/cm$^3$ or less, and further preferably 0.885 g/cm$^3$ or less.

Further, the density of the olefin-based resin composition to be used in the present invention is preferably 0.840 g/cm$^3$ or greater because this reduces blocking from occurring in the porous film of the present invention. The density is more preferably 0.850 g/cm$^3$ or greater, further preferably 0.860 g/cm$^3$ or greater.

To summarize the above, the density of the olefin-based resin composition is preferably 0.840 g/cm$^3$ or greater to less than 0.900 g/cm$^3$, more preferably from 0.850 to 0.895 g/cm$^3$, further preferably from 0.860 to 0.885 g/cm$^3$.

With the aim of setting the density of the olefin-based resin composition within the aforementioned range, it is preferable that the olefin-based resin composition includes a low-melting-point olefin-based resin as the olefin-based resin. The low-melting-point olefin-based resin is used also with the aim of imparting flexibility to the porous film of the present invention. It is preferable that the low-melting-point olefin-based resin is a copolymer of ethylene and an $\alpha$-olefin (also referred to hereinafter as "ethylene-$\alpha$-olefin copolymer"). Examples of $\alpha$-olefins may include propylene, 1-butene, 1-pentene, 1-hexene, etc. Particularly, a copolymer of ethylene and $\alpha$-olefin polymerized with a metallocene catalyst is more preferable because the film strength against tearing, piercing, etc., can be further improved.

From the viewpoint of imparting flexibility to the porous film of the present invention, it is preferable that the melting point of the low-melting-point olefin-based resin is preferably below 90°C, more preferably below 80°C, further preferably below 70°C. From the viewpoint of achieving form stability of the porous film, it is preferable that the melting point of the low-melting-point olefin-based resin is preferably 40°C or higher. The melting point of the low-melting-point olefin-based resin included in the porous film and having a melting point below 90°C can be measured according to the following method. A porous film weighing approximately 2.0 mg is used as a sample. Using a differential scanning calorimeter (DSC7000X from Hitachi High-Tech Science Corporation), differential scanning calorimetry (DSC) is conducted within a measurement temperature range of from 10°C to 260°C at a temperature rise rate of 10°C/min in an atmospheric environment. In the obtained DSC curve, an endothermic peak due to melting of the low-melting-point olefin-based resin is observed in a temperature range below 90°C, and the melting point of the low-melting-point olefin-based resin is the temperature at the apex of the observed endothermic peak.

Note that it is possible to distinguish the melting point of an additive included in the porous film from the melting point of the low-melting-point olefin-based resin by collecting the additive that has bled out from the porous film and measuring the melting point thereof. A method for efficiently collecting an additive from the porous film is as follows.

First, using a Labo Plastomill (from Toyo Seiki Seisaku-Sho, Ltd.), the porous film is kneaded at 160°C at 30 rpm for 10 minutes, to obtain a resin lump.

Next, using a Labo Press (from Toyo Seiki Seisaku-Sho, Ltd.), the resin lump is pressed at 150°C at 13 MPa for 1 minute, and then cold-pressed at ordinary temperature at 13 MPa for 1 minute, to obtain a pressed film having a thickness of approximately 0.5 mm.

Finally, the pressed film is stored in a 50°C environment for 1 week. In this way, a larger amount of additive bleeds out to the surface of the pressed film compared to the state of the porous film, and therefore, the additive can be collected efficiently. Examples of methods for collecting the additive from the surface of the pressed film may include wiping-off with a wipe, scraping with a spatula, etc.

From the viewpoint of imparting even more flexibility to the porous film of the present invention, it is preferable that

the density of the low-melting-point olefin-based resin is preferably 0.895 g/cm$^3$ or less, more preferably 0.885 g/cm$^3$ or less, further preferably 0.875 g/cm$^3$ or less. From the viewpoint of maintaining the strength of the porous film, it is preferable that the density of the low-melting-point olefin-based resin is preferably 0.840 g/cm$^3$ or greater, more preferably 0.850 g/cm$^3$ or greater, further preferably 0.860 g/cm$^3$ or greater.

In order for the low-melting-point olefin-based resin to achieve both the preferable density range and the preferable melting point range, it is preferable that the low-melting-point olefin-based resin is a random copolymer.

From the viewpoint of keeping residual strain after elongational deformation small while imparting satisfactory flexibility to the porous film of the present invention, it is preferable that the olefin-based resin composition to be used in the present invention includes at least 30 parts by mass of the aforementioned low-melting-point olefin-based resin in 100 parts by mass of the olefin-based resin composition. From the viewpoint of making this advantage even more remarkable, it is preferable that the content of the low-melting-point olefin-based resin is more preferably 35 parts by mass or greater, further preferably 40 parts by mass or greater, in 100 parts by mass of the olefin-based resin composition.

Further, it is preferable that the olefin-based resin composition to be used in the present invention contains at most 95 parts by mass of the low-melting-point olefin-based resin in 100 parts by mass of the olefin-based resin composition, because this reduces blocking from occurring in the porous film of the present invention. From the viewpoint of making this advantage even more remarkable, it is preferable that the content of the low-melting-point olefin-based resin is more preferably 92 parts by mass or less, further preferably 90 parts by mass or less, in 100 parts by mass of the olefin-based resin composition.

To summarize the above, it is preferable that, in the olefin-based resin composition to be used in the present invention, the content of the low-melting-point olefin-based resin is preferably from 30 to 95 parts by mass, more preferably from 35 to 92 parts by mass, further preferably from 40 to 90 parts by mass, in 100 parts by mass of the olefin-based resin composition.

In addition to the low-melting-point olefin-based resin, or instead of the low-melting-point olefin-based resin, it is preferable that the olefin-based resin composition to be used in the present invention includes a high-melting-point olefin-based resin from the viewpoint of being able to further impart heat resistance, form stability, and processability to the porous film of the present invention. From the viewpoint of also achieving the porous film's flexibility, it is preferable that the density of the high-melting-point olefin-based resin is relatively low, and more specifically, preferably 0.950 g/cm$^3$ or less, more preferably 0.940 g/cm$^3$ or less, further preferably 0.930 g/cm$^3$ or less.

Further, from the viewpoint of reducing blocking from occurring, it is preferable that the density of the high-melting-point olefin-based resin is preferably 0.900 g/cm$^3$ or greater, more preferably 0.905 g/cm$^3$ or greater, further preferably 0.910 g/cm$^3$ or greater.

To summarize the above, it is preferable that the density of the high-melting-point olefin-based resin is preferably from 0.900 to 0.950 g/cm$^3$, more preferably from 0.905 to 0.940 g/cm$^3$, further preferably from 0.910 to 0.930 g/cm$^3$.

For the high-melting-point olefin-based resin having the aforementioned density, it is preferable to use polyethylene, such as low-density polyethylene or linear low-density polyethylene, and more preferably linear low-density polyethylene, because heat resistance at the time of stretching can be improved and thus stretching can be conducted uniformly. Particularly, a linear low-density polyethylene polymerized with a metallocene catalyst is more preferable because the film strength against tearing, piercing, etc., can be further improved.

A metallocene catalyst is a catalyst in which metallocene-which is a compound having a structure wherein a transition metal, such as titanium, zirconium, hafnium, etc., is sandwiched by unsaturated cyclic compounds containing, for example, a $\pi$ electron-based cyclopentadienyl group or substituted cyclopentadienyl group-is used in combination with a promoter such as an aluminum compound etc. Examples of metallocenes may include titanocene, zirconocene, etc. Examples of aluminum compounds may include alkyl aluminoxanes, alkylaluminums, aluminum halides, alkylaluminum halides, etc.

In the olefin-based resin composition to be used in the present invention, from the viewpoint of being able to further impart heat resistance, form stability, and processability to the porous film, it is preferable that the content of the high-melting-point olefin-based resin having the aforementioned density is 5 parts by mass or greater in 100 parts by mass of the olefin-based resin composition. From the viewpoint of making this advantage even more remarkable, it is preferable that the content of the high-melting-point olefin-based resin is more preferably 8 parts by mass or greater, further preferably 10 parts by mass or greater, in 100 parts by mass of the olefin-based resin composition.

Further, in the olefin-based resin composition to be used in the present invention, from the viewpoint of also achieving the porous film's flexibility, it is preferable that the content of the high-melting-point olefin-based resin is 70 parts by mass or less in 100 parts by mass of the olefin-based resin composition. From the viewpoint of making this advantage even more remarkable, it is preferable that the content of the high-melting-point olefin-based resin is more preferably 65 parts by mass or less, further preferably 60 parts by mass or less, in 100 parts by mass of the olefin-based resin composition.

To summarize the above, in the olefin-based resin composition to be used in the present invention, it is preferable that the content of the high-melting-point olefin-based resin having the aforementioned density is preferably from 5 to 70

parts by mass, more preferably from 8 to 65 parts by mass, further preferably from 10 to 60 parts by mass, in 100 parts by mass of the olefin-based resin composition.

In order to achieve high-speed molding of the porous film which is produced by a melt molding method, it is preferable that the melting point of the high-melting-point olefin-based resin to be used in the present invention is preferably 95°C or higher, more preferably 100°C or higher, further preferably 110°C or higher, to enable solidification in a short time. The melting point of the high-melting-point olefin-based resin included in the porous film can be measured according to the following method. A porous film weighing approximately 2.0 mg is used as a sample. Using a differential scanning calorimeter (DSC7000X from Hitachi High-Tech Science Corporation), differential scanning calorimetry (DSC) is conducted within a measurement temperature range of from 10°C to 260°C at a temperature rise rate of 10°C/min in an atmospheric environment. In the obtained DSC curve, an endothermic peak due to melting of the high-melting-point olefin-based resin is observed in a temperature range of 95°C or higher, and the melting point of the high-melting-point olefin-based resin is the temperature at the apex of the endothermic peak. Note that the method for distinguishing the high-melting-point olefin-based resin from an additive included in the porous film is the same as the aforementioned method for distinguishing the low-melting-point olefin-based resin from an additive included in the porous film.

A preferable embodiment of the present invention is an embodiment wherein the olefin-based resin composition contains: an ethylene-α-olefin copolymer which is a low-melting-point polyethylene resin and polymerized with a metallocene catalyst; and a linear low-density polyethylene which is a high-melting-point polyethylene resin and polymerized with a metallocene catalyst. A porous film according to this embodiment has very high flexibility.

The inorganic filler to be used in the present invention is a substance that causes interfacial peeling with respect to the olefin-based resin composition, to thereby form micropores. From this viewpoint, it is preferable that the average particle size $D_{50}$ of the inorganic filler is preferably 30 μm or less, more preferably 10 μm or less, and preferably 0.5 μm or greater, more preferably 1 μm or greater. The average particle size $D_{50}$ of the inorganic filler refers to weight cumulative particle diameter at a cumulative weight of 50 mass%, as measured by laser diffraction/scattering particle size distribution measurement.

Examples of the inorganic filler may include calcium carbonate, gypsum, talc, clay, kaoline, silica, diatomaceous earth, magnesium carbonate, barium carbonate, magnesium sulfate, barium sulfate, calcium phosphate, aluminum hydroxide, zinc oxide, titanium oxide, alumina, mica, zeolite, carbon black, and mixtures thereof. Particularly, it is preferable to use calcium carbonate because it is easy to make adjustments to achieve the particle size described above.

From the viewpoint of forming a sufficient number of micropores and sufficiently improving the moisture permeability of the porous film, it is preferable that the content of the inorganic filler is preferably 50 parts by mass or greater, more preferably 60 parts by mass or greater, even more preferably 80 parts by mass or greater, with respect to 100 parts by mass of the olefin-based resin composition.

From the viewpoint of sufficiently improving the leakage preventability of the porous film, it is preferable that the content of the inorganic filler is preferably 400 parts by mass or less, more preferably 300 parts by mass or less, even more preferably 200 parts by mass or less, with respect to 100 parts by mass of the olefin-based resin composition.

The porous film of the present invention includes a triglyceride. The triglyceride is blended mainly as a water repellent to improve the water repellency and leakage preventability of the porous film. In the technical field of porous films, there have been techniques of blending triglycerides to porous films, but the triglyceride to be used in the present invention is of a different type from conventional triglycerides that have been used in the present technical field. More specifically, the triglyceride to be used in the present invention includes a group derived from a fatty acid having from 16 to 22 carbon atoms, wherein the group is a hydrocarbon group having no unsaturated bond nor substituent. Inventors' studies have revealed that, by using such a triglyceride, the water repellency of the porous film containing this triglyceride is improved compared to conventional cases, and the leakage preventability of the porous film is also improved compared to conventional cases.

Further, Inventors' studies have revealed that, by including this triglyceride in the porous film, an additional effect is achieved wherein, even in cases where the porous film contains the aforementioned low-melting-point olefin-based resin, blocking between films is further suppressed from occurring. It is known that a film containing a low-melting-point olefin-based resin typically tends to undergo blocking.

From the viewpoint of making these advantages even more remarkable, it is preferable that the amount of the triglyceride to be blended in the porous film of the present invention is preferably 0.1 parts by mass or greater, more preferably 0.5 parts by mass or greater, further preferably 1.0 parts by mass or greater, with respect to 100 parts by mass of the olefin-based resin composition.

From the viewpoint of improving shapeability and reducing deterioration in the porous film's strength, it is preferable that the amount of the triglyceride to be blended is preferably 30 parts by mass or less, more preferably 25 parts by mass or less, further preferably 20 parts by mass or less, with respect to 100 parts by mass of the olefin-based resin composition.

To summarize the above, it is preferable that the amount of the triglyceride to be blended is preferably from 0.1 to 30 parts by mass, more preferably from 0.5 to 25 parts by mass, further preferably from 1.0 to 20 parts by mass, with respect to 100 parts by mass of the olefin-based resin composition.

The triglyceride to be used in the present invention is represented by the following formula (1).

[Chem. 1]

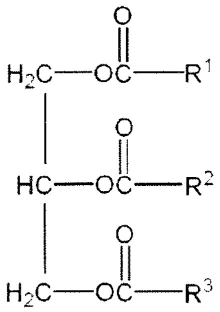

(1)

In the formula, $R^1$ to $R^3$ represent the same hydrocarbon group or different hydrocarbon groups. It is preferable that at least one of $R^1$ to $R^3$ is a group derived from a fatty acid having from 16 to 22 carbon atoms, and this group is a hydrocarbon group including neither an unsaturated bond nor a substituent. Note that an alkyl group of palmitic acid-which is a fatty acid having 16 carbon atoms-has 15 carbon atoms.

A "hydrocarbon group not including an unsaturated bond" refers to a hydrocarbon group including neither a carbon-carbon double bond nor triple bond. In other words, it is an alkyl group. Further, a "hydrocarbon group not including a substituent" means that none of the hydrogen atoms included in the hydrocarbon group is substituted by another atom or atomic group (e.g., hydroxy group). Therefore, a "hydrocarbon group including neither an unsaturated bond nor a substituent" is synonymous with a non-substituted alkyl group.

In the description below, a triglyceride to be used in the present invention may be referred to as "triglyceride of the present invention" for convenience's sake.

From the viewpoint of obtaining a porous film having even higher water repellency, it is preferable that, in the triglyceride represented by formula (1), at least one of $R^1$ to $R^3$ is a group derived from a fatty acid having 18 carbon atoms, and this group is a hydrocarbon group including neither an unsaturated bond nor a substituent.

Further, in the triglyceride represented by formula (1), in cases where one or two of $R^1$ to $R^3$ is/are a group other than a group derived from a fatty acid having from 16 to 22 carbon atoms (which is a hydrocarbon group including neither an unsaturated bond nor a substituent), the type of such group is not particularly limited as long as it is a group derived from a fatty acid, but from the viewpoint of obtaining a porous film having even higher water repellency, it is preferable that this group includes neither an unsaturated bond nor a substituent.

It is preferable that the triglyceride of the present invention is a triglyceride in which the number of carbon atoms in the fatty acid residue has been adjusted. In this way, the water repellency of the present film can be improved. More specifically, the triglyceride is preferably (A) or (B) below.

(A) A triglyceride including a mixture of a triglyceride including, in a single molecule, at least a group derived from a fatty acid having 18 carbon atoms and a triglyceride including, in a single molecule, at least a group derived from a fatty acid having from 16 to 22 carbon atoms (except for a fatty acid having 18 carbon atoms).

(B) A triglyceride including, in a single molecule, at least one group derived from a saturated fatty acid having 18 carbon atoms and at least one group derived from a saturated fatty acid having from 16 to 22 carbon atoms (except for a fatty acid having 18 carbon atoms).

Particularly, from the viewpoint of further improving water repellency, it is preferable that the triglyceride includes, in a single molecule, at least one group derived from a saturated fatty acid having 18 carbon atoms and at least one group derived from a saturated fatty acid having from 16 to 22 carbon atoms (except for a fatty acid having 18 carbon atoms).

Concrete examples of combinations of a fatty acid having 18 carbon atoms and a fatty acid having 16 carbon atoms may be (C) or (D) below.

(C) A triglyceride including a mixture of a triglyceride including, in a single molecule, at least a group derived from a fatty acid having 18 carbon atoms (i.e., stearic acid) (this group is a hydrocarbon group including neither an unsaturated bond nor a substituent) and a triglyceride including, in a single molecule, at least a group derived from a fatty acid having 16 carbon atoms (i.e., palmitic acid) (this group is a hydrocarbon group including neither an unsaturated bond nor a substituent).

(D) A triglyceride including, in a single molecule, at least one group derived from a fatty acid having 18 carbon atoms (this group is a hydrocarbon group including neither an unsaturated bond nor a substituent) and at least one group derived from a fatty acid having 16 carbon atoms (this group is a hydrocarbon group including neither an unsaturated bond

nor a substituent).

In cases where the triglyceride of the present invention includes a plurality of types of triglycerides as in (C), it is preferable that at least one type of triglyceride includes, in a single molecule, at least one group derived from a saturated fatty acid having 16 carbon atoms (this triglyceride is also referred to as "triglyceride 16"). Triglyceride 16 may include, in a single molecule, one group derived from a saturated fatty acid having 16 carbon atoms (this triglyceride is also referred to as "triglyceride P"), two of these groups (this triglyceride is also referred to as "triglyceride PP"), or three of these groups (this triglyceride is also referred to as "triglyceride PPP").

Note that the types of the rest of the fatty acid residues in triglyceride P and triglyceride PP are not particularly limited, and they may be, for example, residues of saturated fatty acids having from 12 to 24 carbon atoms.

Triglyceride 16 may be constituted only by triglyceride P, or only by triglyceride PP, or only by triglyceride PPP.

Triglyceride 16 may be a combination of two or more types selected from triglyceride P, triglyceride PP, and triglyceride PPP. For example, triglyceride 16 may be a combination of triglyceride P and triglyceride PP, a combination of triglyceride P and triglyceride PPP, a combination of triglyceride PP and triglyceride PPP, or a combination of triglyceride P, triglyceride PP, and triglyceride PPP.

In cases where the triglyceride of the present invention includes a plurality of types of triglycerides as in (C), it is also preferable that at least one type of triglyceride includes, in a single molecule, at least one group derived from a saturated fatty acid having 18 carbon atoms (this triglyceride is also referred to as "triglyceride 18"). Triglyceride 18 may include, in a single molecule, one group derived from a fatty acid having 18 carbon atoms (this triglyceride is also referred to as "triglyceride S"), two of these groups (this triglyceride is also referred to as "triglyceride SS"), or three of these groups (this triglyceride is also referred to as "triglyceride SSS").

Note that the types of the rest of the fatty acid residues in triglyceride S and triglyceride SS are not particularly limited, and they may be, for example, residues of saturated fatty acids having from 12 to 24 carbon atoms.

Triglyceride 18 may be constituted only by triglyceride S, or only by triglyceride SS, or only by triglyceride SSS.

Triglyceride 18 may be a combination of two or more types selected from triglyceride S, triglyceride SS, and triglyceride SSS. For example, triglyceride 18 may be a combination of triglyceride S and triglyceride SS, a combination of triglyceride S and triglyceride SSS, a combination of triglyceride SS and triglyceride SSS, or a combination of triglyceride S, triglyceride SS, and triglyceride SSS.

In case of (C), the triglyceride of the present invention may be constituted only by triglyceride 16 and triglyceride 18, or may include other triglycerides in addition to triglyceride 16 and triglyceride 18. Examples of other triglycerides may include triglycerides that do not include any groups derived from fatty acids having from 16 to 22 carbon atoms at all, and triglycerides including groups derived from fatty acids having from 16 to 22 carbon atoms (except for triglyceride 16 and triglyceride 18).

Where a group derived from a saturated fatty acid having 16 carbon atoms is defined as "P", a group derived from a saturated fatty acid having 18 carbon atoms is defined as "S", and groups derived from fatty acids other than a saturated fatty acid including 16 carbon atoms and a saturated fatty acid including 18 carbon atoms are defined respectively as "X" and "Y", examples of combinations of aliphatic groups constituting the triglyceride of the present invention may include PPP, SSS, PPX, SSX, PXY, SXY, PPS, PSS, and PSX. Triglyceride structures represented by PPX, SSX, PXY, SXY, and PSX are (a) to (m) below. Note that, although the structures of PPS and PSS are not illustrated, the structure of PPS is similar to the structure of PPX, and the structure of PSS is similar to the structure of SSX.

[Chem. 2]

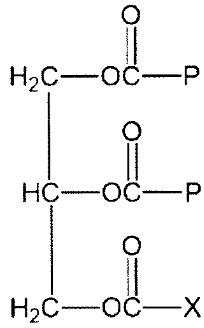 (a) 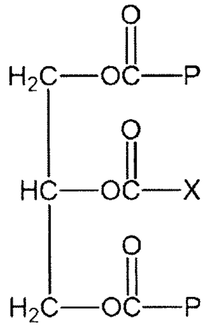 (b)

[Chem. 3]

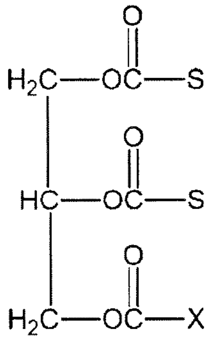 (c) 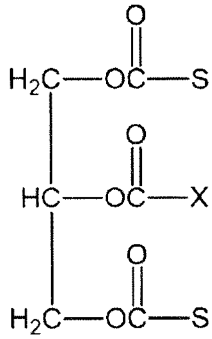 (d)

[Chem. 4]

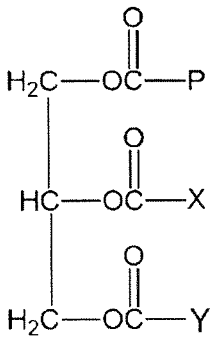 (e) 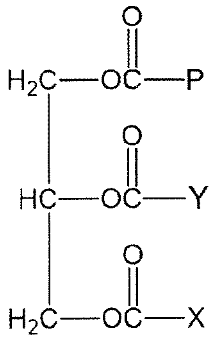 (f) 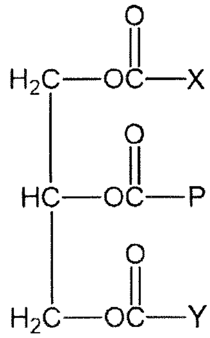 (g)

[Chem. 5]

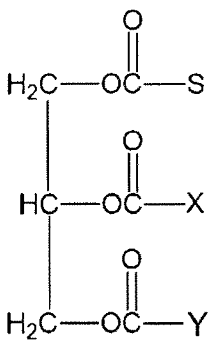 (h) 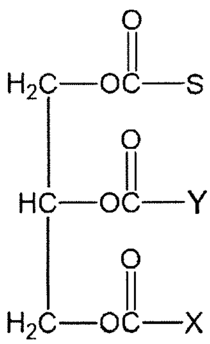 (i) 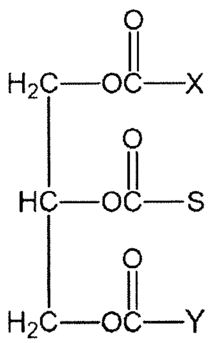 (j)

[Chem. 6]

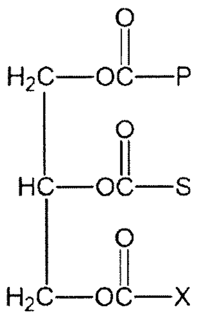 (k) 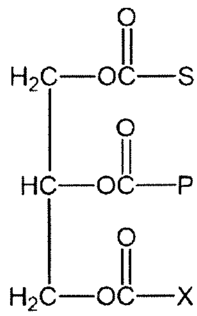 (l) 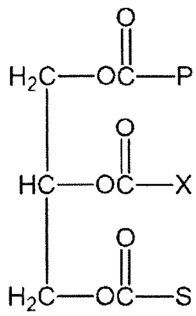 (m)

For the triglyceride of the present invention, one type of the various triglycerides described above may be used singly. For example, in case of (D), the triglyceride of the present invention may be constituted by a triglyceride including, in a single molecule, at least one group derived from a saturated fatty acid having 16 carbon atoms, at least one group derived from a saturated fatty acid having 18 carbon atoms, and no group derived from a fatty acid other than the above. Alternatively, the triglyceride of the present invention may be constituted by a triglyceride including one group derived from a saturated fatty acid having 16 carbon atoms, one group derived from a saturated fatty acid having 18 carbon atoms, and one group derived from another fatty acid.

The triglyceride of the present invention may be a combination of two or more types of the various triglycerides described above. For example, the triglyceride of the present invention may be a combination of (C) and (D) described above. Alternatively, the triglyceride may be a combination of two or more types of (D).

Further, the triglyceride of the present invention may be a combination of one or more types of the triglycerides described above and other types of triglycerides. Examples of such other types of triglycerides may include triglycerides (except for triglyceride 16 and triglyceride 18) including groups derived from fatty acids having from 14 to 22 carbon atoms.

In the present invention, from the viewpoint of further improving the water repellency of the porous film of the present invention, it is preferable to use one type of the various triglycerides described above singly, or use a combination of two or more types of only the various triglycerides described above.

From the viewpoint of obtaining a porous film having even higher water repellency, it is preferable that, in the triglyceride included in the porous film of the present invention, from 28 to 96 mass%, preferably from 28 to 70 mass%, more preferably from 29 to 67 mass%, even more preferably from 30 to 64 mass%, with respect to the total amount of groups derived from fatty acids included in all the triglyceride is a group derived from a fatty acid having 18 carbon atoms (this group is a hydrocarbon group including neither an unsaturated bond nor a substituent).

From the viewpoint of further improving the water repellency of the porous film and from the viewpoint of reducing the amount of time required until the film exhibits water repellency, in the triglyceride included in the porous film of the present invention, it is preferable that, with respect to the total amount of groups derived from fatty acids included in all the triglyceride, from 28 to 68 mass% is a group derived from a fatty acid having 18 carbon atoms (this group is a hydrocarbon group including neither an unsaturated bond nor a substituent), and from 26 to 70 mass% is a group derived from a fatty acid having 16 carbon atoms (this group is a hydrocarbon group including neither an unsaturated bond nor a substituent). Note that the total of the percentage of the group derived from a fatty acid having 18 carbon atoms and the percentage of the group derived from a fatty acid having 16 carbon atoms does not exceed 100 mass%.

In this case, the percentage of the group derived from a fatty acid having 18 carbon atoms is more preferably from 29 to 66 mass%, further preferably from 30 to 64 mass%.

On the other hand, the percentage of the group derived from a fatty acid having 16 carbon atoms is more preferably from 27 to 69 mass%, further preferably from 28 to 68 mass%. When the percentage of the group derived from a fatty acid having 16 carbon atoms is higher, the triglyceride easily precipitates out onto the surface of the porous film, and the amount of time required until the film exhibits water repellency is reduced.

From the viewpoint of improving the thermal stability of the porous film and from the viewpoint of further improving the water repellency of the porous film, in the triglyceride included in the porous film of the present invention, it is preferable that, with respect to the total amount of groups derived from fatty acids included in all the triglyceride, from 28 to 47 mass% is a group derived from a fatty acid having 18 carbon atoms (this group is a hydrocarbon group including neither an unsaturated bond nor a substituent), and from 40 to 60 mass% is a group derived from a fatty acid having 22 carbon atoms (this group is a hydrocarbon group including neither an unsaturated bond nor a substituent). Note that the total of the percentage of the group derived from a fatty acid having 18 carbon atoms and the percentage of the group derived from a fatty acid having 22 carbon atoms does not exceed 100 mass%.

In this case, the percentage of the group derived from a fatty acid having 18 carbon atoms is more preferably from

30 to 45 mass%, further preferably from 32 to 43 mass%.

On the other hand, the percentage of the group derived from a fatty acid having 22 carbon atoms is more preferably from 42 to 58 mass%, further preferably from 44 to 56 mass%. When the percentage of the group derived from a fatty acid having 22 carbon atoms is higher, the melting point of the triglyceride becomes higher, and the thermal stability of molded products is improved. Also, it is possible to reduce roller contamination in processing machines.

The respective percentages of the group derived from a fatty acid having 16 carbon atoms, the group derived from a fatty acid having 18 carbon atoms, and the group derived from a fatty acid having 22 carbon atoms, with reference to the total amount of groups derived from fatty acids included in all the triglyceride(s), can be measured according to the following method.

The triglyceride that has bled out onto the surface of the film is wiped off and collected with a cellulose wiper.

The ester bonds in the obtained triglyceride are hydrolyzed with an alkali, and the methyl-esterified fatty acids are subjected to quantitative analysis by gas chromatography.

Whether or not a triglyceride includes alkyl chains having different numbers of carbon atoms in a single molecule can be determined by time-of-flight mass spectrometry (TOF-MS). More specifically, the molecular weight distribution of the triglyceride is measured by TOF-MS, to determine, from the molecular weight of a single molecule, whether or not the molecule includes alkyl groups having different numbers of carbon atoms. For compounds having the same molecular weight, whether or not a single molecule includes alkyl chains having different numbers of carbon atoms can be determined by using a tandem mass spectrometer (MS/MS) for the mass spectrometer. Specific ions are selected in the first mass analyzer and undergo collision with inert gas, and the generated fragment ions are separated and detected in the second mass analyzer.

From the viewpoint of further improving the water repellency of the porous film of the present invention, it is preferable that the triglyceride of the present invention does not include groups derived from unsaturated fatty acids. The expression "does not include groups derived from unsaturated fatty acids" encompasses both cases where the triglyceride includes absolutely no group derived from an unsaturated fatty acid at all and cases where a small amount of unsaturated fatty acid is inevitably included. "Cases where a small amount of unsaturated fatty acid is inevitably included" refer, for example, to cases in which the percentage of groups derived from unsaturated fatty acids is 2 mass% or less with reference to the total amount of groups derived from fatty acids and included in all the triglyceride(s) contained in the porous film.

Similar to the above, from the viewpoint of further improving the water repellency of the porous film of the present invention, it is preferable that the triglyceride of the present invention does not include groups derived from fatty acids having a hydroxy group. A "fatty acid having a hydroxy group" refers to a fatty acid in which at least one hydrogen atom in the hydrocarbon group of the fatty acid has been substituted by a hydroxy group. The expression "does not include groups derived from fatty acids having a hydroxy group" encompasses both cases where the triglyceride includes absolutely no group derived from a fatty acid having a hydroxy group at all and cases where a small amount of groups derived from fatty acids having a hydroxy group is inevitably included. "Cases where a small amount of groups derived from fatty acids having a hydroxy group is inevitably included" refer, for example, to cases in which the percentage of groups derived from fatty acids having a hydroxy group is 2 mass% or less with reference to the total amount of groups derived from fatty acids and included in all the triglyceride(s) contained in the porous film.

From the viewpoint of further improving the water repellency of the porous film of the present invention, in the porous film of the present invention, it is preferable that the groups derived from fatty acids included in all the triglyceride(s) are hydrocarbon groups not including an unsaturated bond. It is also preferable that the groups derived from fatty acids included in all the triglyceride(s) are hydrocarbon groups not including a substituent.

In the porous film of the present invention, only triglycerides may be included as glycerides, or monoglycerides and/or diglycerides may also be included, in addition to triglycerides, to the extent that the intended effects of the present invention can be achieved.

The porous film of the present invention may include additives separate from the aforementioned triglyceride. Additives to be used herein may be additives capable of imparting various additional properties to the porous film. Examples of such additives may include dispersing agents for the inorganic filler, plasticizers, etc. Note that "additive" does not encompass the aforementioned triglyceride.

As for dispersing agents, it is possible to preferably use agents capable of hydrophobizing the surface of the inorganic filler. From this viewpoint, for the dispersing agent, it is preferable to use a fatty acid, for example. Examples of fatty acids may include caprylic acid, palmitic acid, stearic acid, capric acid, oleic acid, myristic acid, lauric acid, etc.

A plasticizer is used with the aim of imparting flexibility or elasticity to the porous film of the present invention and preventing the porous film of the present invention from making a rustling noise. Preferably usable examples of plasticizers may include monoesters, polyesters, ethylene-$\alpha$-olefin cooligomers, low-molecular-weight polyethylene, olefin oligomers, liquid polyisoprene, liquid polybutadiene, etc.

A monoester is a compound obtained from a monobasic acid and a monovalent alcohol.

On the other hand, a polyester is a compound obtained as a combination of either a polybasic acid and a monovalent alcohol, a monobasic acid and a polyol, or a polybasic acid and a polyol.

An ethylene-α-olefin cooligomer is a low-molecular-weight copolymer of ethylene and an α-olefin such as propylene, 1-butene, 1-pentene, 1-hexene, etc.

Preferably usable examples of the aforementioned monobasic acids, polybasic acids, monovalent alcohols, and polyols may include the following.

Examples of monobasic acids may include monocarboxylic acids of long-chain hydrocarbons having from 10 to 22 carbon atoms.

Examples of polybasic acids may include dicarboxylic acids, tricarboxylic acids, tetracarboxylic acids, etc.

Examples of monovalent alcohols may include monoalcohols of long-chain hydrocarbons having from 10 to 22 carbon atoms.

Examples of polyols may include diols, trimethylol propane, pentaerythritol, dipentaerythritol, sorbitol, sucrose, etc.

Examples of preferable polyesters may include: polyesters in which the carboxylic acid groups or alcohol groups on both terminals of a polyester of diethylene glycol and dimer acid are partially or entirely blocked by stearyl alcohol or stearic acid; polyester of 1,3-butane diol and adipic acid; hexaester of trimethylolpropane, adipic acid and stearic acid; octaester of pentaerythritol, adipic acid and stearic acid; dodecaester of dipentaerythritol, adipic acid and stearic acid; and the like.

On the other hand, examples of preferable monoesters may include esters having a total of 30 or more carbon atoms, obtained by causing dehydration between a monocarboxylic acid having from 1 to 40 carbon atoms and a monoalcohol having from 1 to 40 carbon atoms. Among the above, esters having a total of 30 or more carbon atoms obtained from a monocarboxylic acid and a monoalcohol are preferable, and monoesters having 38 or more carbon atoms and having a branched chain are more preferable. Concrete examples may include isodecyl stearate, isodecyl behenate, isotridecyl stearate, 2-octadecyl stearate, 2-decyltetradecyl laurate, 2-decyltetradecyl stearate, 2-octadecyl behenate, stearyl isostearate, an ester of stearic acid and C20 guerbet alcohol, esters of α-branched fatty acids (having from 18 to 40 carbon atoms) and monoalcohols (having from 6 to 36 carbon atoms), and the like.

In addition to the aforementioned additives, the moisture-permeable film may further include a micropore-forming accelerator. A micropore-forming accelerator is used with the aim of smoothly forming micropores by stretching a resin composition containing a resin and an inorganic filler. As described above, from the viewpoint of improving the flexibility of the moisture-permeable film, it is preferable to include a low-melting-point olefin-based resin in the moisture-permeable film, but since the low-melting-point olefin-based resin is a resin that is relatively less likely to undergo interfacial peeling with respect to the inorganic filler, the use of a micropore-forming accelerator as a material of the moisture-permeable film in addition to the low-melting-point olefin-based resin can promote the aforementioned interfacial peeling.

A preferably usable example of the micropore-forming accelerator is a substance known as a mold-release agent used between metal and resin. Concrete examples may include metal soaps, silicone, fluorocarbon resin, fatty acid amides, hydrocarbon paraffin waxes, etc. Particularly, from the viewpoint of enabling micropore formation even more smoothly, it is preferable to use a metal soap.

For the metal soap, it is possible to suitably use a metal salt of a fatty acid. Examples of fatty acids may include caprylic acid, palmitic acid, stearic acid, capric acid, oleic acid, myristic acid, lauric acid, etc. Examples of metal salts may include salts between these fatty acids and metals such as calcium, aluminum, magnesium, zinc, etc.

From the viewpoint of sufficiently improving the flexibility of the porous film and the dispersibility of the inorganic filler, it is preferable that the amount of additive to be blended, with respect to 100 parts by mass of the olefin-based resin composition, is preferably 0.01 parts by mass or greater, more preferably 1.0 part by mass or greater, even more preferably 2.0 parts by mass or greater.

From the viewpoint of improving shapeability and reducing deterioration in the porous film's strength, it is preferable that the amount of additive to be blended, with respect to 100 parts by mass of the olefin-based resin composition, is preferably 20 parts by mass or less, more preferably 18 parts by mass or less, even more preferably 16 parts by mass or less.

The additive may at least include a first additive whose difference in SP value from the triglyceride is less than 0.37. From the viewpoint of allowing the additive to be blended without inhibiting water repellency, which is brought about by the triglyceride of the present invention, it is preferable that the content of the first additive is 3.5 parts by mass or less with respect to 100 parts by mass in total of the olefin-based resin composition, the inorganic filler, and the triglyceride. From the viewpoint of improving leakage preventability without impairing water repellency, it is preferable that the content of the first additive is preferably from 0.01 to 3.5 parts by mass, more preferably from 0.03 to 3.3 parts by mass, further preferably from 0.06 to 3.2 parts by mass, with respect to 100 parts by mass in total of the olefin-based resin composition, the inorganic filler, and the triglyceride.

Further, the additive may at least include a second additive whose difference in SP value from the triglyceride is 0.37 or greater. From the viewpoint of allowing the additive to be blended without inhibiting water repellency, which is brought about by the triglyceride of the present invention, it is preferable that the content of the second additive is preferably 10 parts by mass or less with respect to 100 parts by mass in total of the olefin-based resin composition, the inorganic filler,

and the triglyceride. In cases where the second additive is included as the additive, from the viewpoint of improving leakage preventability without impairing water repellency, it is preferable that the content of the second additive is preferably from 0.01 to 10 parts by mass, more preferably from 1 to 8 parts by mass, further preferably from 1.5 to 6 parts by mass, with respect to 100 parts by mass in total of the olefin-based resin composition, the inorganic filler, and the triglyceride.

As described above, in the technical field of porous films, there have been techniques for blending triglycerides to porous films. However, Inventors' studies have revealed that randomly blending an additive to a porous film including a triglyceride gives rise to a phenomenon in which water repellency caused by the triglyceride is inhibited. In contrast, when the first additive is used in an amount that is 3.5 parts by mass or less with respect to 100 parts by mass in total of the olefin-based resin composition, the inorganic filler, and the triglyceride, or the second additive is used in an amount that is 10 parts by mass or less with respect to 100 parts by mass in total of the olefin-based resin composition, the inorganic filler, and the triglyceride, it is possible to blend the additive without inhibiting water repellency, which is brought about by the triglyceride of the present invention. From the viewpoint of making this advantage even more remarkable, it is preferable that the difference in SP value of the second additive from the triglyceride of the present invention is preferably 0.50 or greater, further preferably 0.60 or greater. Also, it is preferable that this difference is preferably 4.00 or less, more preferably 3.00 or less, further preferably 1.50 or less.

"SP value" is a physical property value called solubility parameter. Two types of substances with close SP values have easily miscible properties. In other words, two types of substances with close SP values have high mutual solubility. Therefore, when comparing the SP value of the triglyceride of the present invention and the SP value of an additive, the SP value of the triglyceride of the present invention may be greater, or the SP value of the additive may be greater. From the viewpoint of reducing inhibition, caused by the additive, in water repellency of the triglyceride of the present invention, it is preferable that the SP value of the triglyceride is greater than the SP value of the additive.

Examples of the first additive selected such that the difference in SP value from the triglyceride of the present invention is less than 0.37 may include some of the fatty acids, alcohols, monoesters, polyesters, and metal soaps given as examples of the aforementioned dispersing agents, plasticizers, and micropore-forming accelerators.

Examples of the second additive selected such that the difference in SP value from the triglyceride of the present invention is 0.37 or greater may include olefin oligomers, ethylene-$\alpha$-olefin cooligomers, low-molecular-weight polyethylene, silicone rubber, fluorocarbon rubber, fluorocarbon resin, polystyrene, etc. In the present invention, only the second additive may be used as the additive, or both the second additive and the first additive may be used in combination. In some cases, only the first additive may be used.

The SP value is calculated according to Fedors' method [R.F. Fedors, Polym. Eng. Sci., 14, 147 (1974)], and the unit is expressed as $(cal/cm^3)^{1/2}$.

In cases where the porous film includes a plurality of types of the triglycerides of the present invention, the following SP value $\delta mix$ of a mixture is calculated.

$$\delta mix = \Sigma \delta i \varphi i \ (cal/cm^3)^{1/2}$$

In the equation, $\delta i$ represents the SP value of each component constituting the mixture, and $\varphi i$ represents the volume fraction of that component.

The porous film of the present invention can be obtained from a material composition including an olefin-based resin composition, an inorganic filler, a triglyceride, and an additive, by melting and molding the material composition into a resin sheet, and stretching the resin sheet at least in a uniaxial direction.

For example, the porous film of the present invention can be manufactured efficiently by the following method.

First, each of the components constituting the aforementioned material composition is preliminarily mixed using, for example, a Henschel mixer or a Super mixer, and then the composition is mixed/kneaded and pelletized with a uniaxial or biaxial extruder. Next, the obtained pellets are used to form a film with a molding machine, to thereby obtain a resin sheet. For example, a T-die-type or inflation-type molding machine may be used.

By subjecting this resin sheet to uniaxial or biaxial stretching, interfacial peeling occurs between the olefin-based resin composition and the inorganic filler, which makes the resin sheet porous. For this stretching, techniques such as roll stretching, with which the sheet can be stretched in the machine direction, or tentering, with which the sheet can be stretched also in the traverse direction in addition to the machine direction, may be employed. In this way, the porous film of the present invention is obtained. It is preferable that the resin sheet is stretched at least in a uniaxial direction preferably to at least 1.1 times, more preferably to at least 1.5 times, further preferably to at least 2 times, so that the area increases as the sheet is stretched. From the viewpoint of avoiding deterioration in tear strength associated with excessive molecular orientation caused by excessive stretching, it is preferable that the sheet is stretched preferably to at most 5.0 times, more preferably to at most 4.5 times, further preferably to at most 4 times.

Depending on the application, the basis weight of the porous film of the present invention may be, for example, from around 5 to 100 $g/m^2$. Further, depending on the application, the thickness of the porous film of the present invention

may be, for example, from around 4 to 90 μm.

The porous film of the present invention is suitable for use in applications requiring not only moisture permeability, but also air permeability and leakage preventability. For example, the porous film of the present invention is applicable to leak-proof sheets for absorbent articles, such as disposable diapers, sanitary napkins, etc., and waterproof sheets for rain gear etc.

The porous film of the present invention is particularly useful as a constituent member of an absorbent article. The present invention encompasses an absorbent article including the aforementioned porous film of the present invention.

An absorbent article of the present invention typically includes: a topsheet forming a skin-facing surface; a leak-proof sheet forming a non-skin-facing surface; and a liquid-retentive absorbent member arranged between these two sheets. The absorbent article may further include leak-proof cuffs respectively on the skin-facing surface's both lateral sides along the longitudinal direction.

The topsheet is typically liquid-permeable.

The absorbent member typically includes an absorbent core and a core-wrap sheet that wraps the absorbent core.

It is preferable to use the porous film of the present invention as a leak-proof sheet or a leak-proof cuff because this is particularly effective.

In cases of using the porous film of the present invention as a leak-proof sheet or a leak-proof cuff for an absorbent article, the porous film itself may be used as a leak-proof sheet, or the porous film may be used in combination with another sheet material such as a nonwoven fabric.

Note that the "skin-facing surface" is the surface of the absorbent article, or its constituent members (e.g., absorbent core), that faces the wearer's skin side when the absorbent article is worn-i.e., the surface to be arranged relatively closer to the wearer's skin-whereas the "non-skin-facing surface" is the surface of the absorbent article, or its constituent members, that faces the opposite side from the skin side when the absorbent article is worn-i.e., the surface relatively farther from the wearer's skin.

For the topsheet, absorbent core, and core-wrap sheet, any component ordinarily used in this type of absorbent article may be used without particular limitation.

The absorbent article of the present invention broadly encompasses articles usable for absorbing body fluid (urine, soft feces, menstrual blood, sweat, etc.) discharged from the human body, with examples including disposable diapers, sanitary napkins, sanitary underpants, incontinence pads, etc.

In relation to the aforementioned embodiments, the present invention further discloses the following porous films.

{1} A porous film comprising at least an olefin-based resin composition, an inorganic filler, and a triglyceride, wherein:

a content of the filler is from 50 to 400 parts by mass with respect to 100 parts by mass of the olefin-based resin composition;
a content of the triglyceride is from 0.1 to 30 parts by mass with respect to 100 parts by mass of the olefin-based resin composition; and
the triglyceride includes a group derived from a fatty acid having from 16 to 22 carbon atoms, the group being a hydrocarbon group having neither an unsaturated bond nor a substituent.

{2} The porous film as set forth in clause {1}, wherein the triglyceride is

(A) a mixture of a triglyceride including, in a single molecule, at least a group derived from a fatty acid having 18 carbon atoms and a triglyceride including, in a single molecule, at least a group derived from a fatty acid having from 16 to 22 carbon atoms (except for a fatty acid having 18 carbon atoms), or
(B) a triglyceride including, in a single molecule, at least one group derived from a saturated fatty acid having 18 carbon atoms and at least one group derived from a saturated fatty acid having from 16 to 22 carbon atoms (except for a fatty acid having 18 carbon atoms).

{3} The porous film as set forth in clause {1} or {2}, wherein the triglyceride is a triglyceride including, in a single molecule, at least one group derived from a saturated fatty acid having 18 carbon atoms and at least one group derived from a saturated fatty acid having from 16 to 22 carbon atoms (except for a fatty acid having 18 carbon atoms).
{4} The porous film as set forth in any one of clauses {1} to {3}, wherein, with respect to a total amount of groups derived from fatty acids included in all the triglyceride, from 28 to 96 mass% is a group derived from a fatty acid having 18 carbon atoms.
{5} The porous film as set forth in any one of clauses {1} to {4}, wherein, with respect to the total amount of groups derived from fatty acids included in all the triglyceride, from 28 to 70 mass% is a group derived from a fatty acid having 18 carbon atoms.

{6} The porous film as set forth in any one of clauses {1} to {5}, wherein, with respect to the total amount of groups derived from fatty acids included in all the triglyceride, from 28 to 68 mass% is a group derived from a fatty acid having 18 carbon atoms and from 26 to 70 mass% is a group derived from a fatty acid having 16 carbon atoms.
{7} The porous film as set forth in any one of clauses {1} to {5}, wherein, with respect to the total amount of groups derived from fatty acids included in all the triglyceride, from 28 to 47 mass% is a group derived from a fatty acid having 18 carbon atoms and from 40 to 60 mass% is a group derived from a fatty acid having 22 carbon atoms.
{8} The porous film as set forth in any one of clauses {1} to {7}, wherein the groups derived from fatty acids included in all the triglyceride are hydrocarbon groups not having an unsaturated bond.
{9} The porous film as set forth in any one of clauses {1} to {8}, wherein the groups derived from fatty acids included in all the triglyceride are hydrocarbon groups not having a substituent.
{10} The porous film as set forth in any one of clauses {1} to {9}, wherein:

the olefin-based resin composition has a density of less than 0.900 g/cm$^3$; and
the olefin-based resin composition has a density of 0.840 g/cm$^3$ or greater.

{11} The porous film as set forth in any one of clauses {1} to {10}, wherein:

the olefin-based resin composition includes a low-melting-point olefin-based resin having a melting point below 90°C;
the low-melting-point olefin-based resin has a density of 0.895 g/cm$^3$ or less; and
the low-melting-point olefin-based resin has a density of 0.840 g/cm$^3$ or greater.

{12} The porous film as set forth in clause {11}, including from 30 to 95 parts by mass of the low-melting-point olefin-based resin in 100 parts by mass of the olefin-based resin composition.
{13} The porous film as set forth in any one of clauses {1} to {12}, wherein:

the olefin-based resin composition includes a high-melting-point olefin-based resin having a melting point of 95°C or higher; and
the high-melting-point olefin-based resin has a density of from 0.900 to 0.950 g/cm$^3$.

{14} The porous film as set forth in clause {13}, wherein the high-melting-point olefin-based resin includes polyethylene.
{15} The porous film as set forth in clause {14}, wherein the high-melting-point olefin-based resin includes a linear low-density polyethylene.
{16} The porous film as set forth in clause {15}, wherein the linear low-density polyethylene includes a linear low-density polyethylene polymerized with a metallocene catalyst.
{17} The porous film as set forth in clause {11} or {12}, wherein the low-melting-point olefin-based resin includes an ethylene-α-olefin copolymer.
{18} The porous film as set forth in clause {17}, wherein the ethylene-α-olefin copolymer includes an ethylene-α-olefin copolymer polymerized with a metallocene catalyst.
{19} The porous film as set forth in any one of clauses {1} to {10}, wherein:

the olefin-based resin composition includes a low-melting-point olefin-based resin having a melting point below 90°C and a high-melting-point olefin-based resin having a melting point of 95°C or higher;
the high-melting-point olefin-based resin includes a linear low-density polyethylene polymerized with a metallocene catalyst; and
the low-melting-point olefin-based resin includes an ethylene-α-olefin copolymer polymerized with a metallocene catalyst.

{20} The porous film as set forth in any one of clauses {1} to {19}, further including an additive, wherein:

the additive includes at least a first additive whose difference in SP value from the triglyceride is less than 0.37; and
a content of the first additive is from 0.01 to 3.5 parts by mass with respect to 100 parts by mass in total of the olefin-based resin composition, the inorganic filler, and the triglyceride.

{21} The porous film as set forth in any one of clauses {1} to {20}, further including an additive, wherein:

the additive includes at least a second additive whose difference in SP value from the triglyceride is 0.37 or greater; and
a content of the second additive is from 0.01 to 10 parts by mass with respect to 100 parts by mass in total of the olefin-based resin composition, the inorganic filler, and the triglyceride.

{22} The porous film as set forth in clause {21}, wherein the difference in SP value of the second additive from the triglyceride is from 0.37 to 4.00.
{23} The porous film as set forth in any one of clauses {1} to {22}, wherein the inorganic filler has an average particle size of 30 μm or less.
{24} The porous film as set forth in any one of clauses {1} to {23}, wherein calcium carbonate is used as the inorganic filler.
{25} The porous film as set forth in any one of clauses {1} to {24}, wherein a content of the inorganic filler is from 60 to 300 parts by mass with respect to 100 parts by mass of the olefin-based resin composition.
{26} The porous film as set forth in any one of clauses {1} to {25}, wherein an amount of the triglyceride to be blended is from 0.1 to 20 parts by mass with respect to 100 parts by mass of the olefin-based resin composition.
{27} The porous film as set forth in any one of clauses {1} to {26}, wherein the porous film is for use in an absorbent article.
{28} The porous film as set forth in clause {27}, wherein the porous film is for use as a leak-proof sheet in the absorbent article.
{29} The porous film as set forth in clause {27}, wherein the porous film is for use as a leak-proof cuff in the absorbent article.
{30} An absorbent article including the porous film as set forth in any one of clauses {1} to {29}.
{31} The absorbent article as set forth in clause {30}, comprising:

a topsheet forming a skin-facing surface;
a leak-proof sheet forming a non-skin-facing surface; and
a liquid-retentive absorbent member arranged between the topsheet and the leak-proof sheet, wherein:
the leak-proof sheet is the porous film.

{32} The absorbent article as set forth in clause {30}, comprising:

a topsheet forming a skin-facing surface;
a leak-proof sheet forming a non-skin-facing surface; and
a liquid-retentive absorbent member arranged between the topsheet and the
leak-proof sheet, wherein:

leak-proof cuffs are provided respectively on the skin-facing surface's both lateral sides along a longitudinal direction; and
the leak-proof cuff is the porous film.

Examples

The present invention is described in further detail below according to Examples. Note, however, that the scope of the present invention is not limited to these Examples. Hereinbelow, "%" and "parts" refer to "mass%" and "parts by mass", respectively, unless specifically stated otherwise.

{Reference Examples 1 to 15 and Comparative Reference Examples 1 to 8}

In the present Reference Examples and Comparative Reference Examples, the water repellency of triglycerides was evaluated according to the following method, employing triglyceride-containing films (non-porous films) as subjects. The results are shown in Tables 1 and 2 below.

(1) Production of Compound:
The components shown in Tables 1 and 2 below were measured in amounts shown in the Tables. The components were placed in a Labo Plastomill (from Toyo Seiki Seisaku-Sho, Ltd.) and mixed and kneaded at 160°C at 30 rpm for 10 minutes, to obtain a compound.

Details of the components shown in Tables 1 and 2 are shown in Tables 3 and 4.

The unit of the compositional makeup shown in Tables 1 and 2 is in "mass%".

(2) Production of Film:
Using a Labo Press (from Toyo Seiki Seisaku-Sho, Ltd.), the compound was pressed at 150°C at 13 MPa for 1 minute. Next, this was cold-pressed at ordinary temperature at 13 MPa for 1 minute, to produce a film (resin sheet). At this time, adjustments were made to obtain a film having a thickness of 0.5 mm.
(3) Evaluation of Water Repellency:
The produced film was immersed in 50 mL of toluene solution, and then, the film surface was wiped and cleaned with tissue paper. The cleaned film was placed in an electric dryer and stored at 40°C for 7 days. After storage, the contact angle on the surface of the film, whose water repellency had been adjusted by bleed-out of triglyceride(s), was measured using a contact angle meter (Drop Master 500 from Kyowa Interface Science Co., Ltd.). Two types of evaluation solutions, respectively having a surface tension of 35.0 mN/m and a surface tension of 44.0 mN/m, were used.

- Measurement method: Sessile drop method
- Evaluation solutions: Wetting tension test mixtures (surface tension at 25°C: 35.0 mN/m and 44.0 mN/m; from Kanto Chemical Co., Inc.)
- Drop amount: 2 μL
- The contact angle after 10 seconds from droplet landing was measured at 5 points per test piece, and the average value of 5 droplets was found as the contact angle value.

[Table 1]

| | | Reference Example | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| High-melting-point olefin-based resin A | | 95 | 95 | 95 | 95 | 95 | 95 | 95 | 95 | 95 | 39.4 | 35.6 | 35.6 | 35.6 | 35.6 | 35.6 |
| Inorganic filler | | - | - | - | - | - | - | - | - | - | 54.8 | 54.8 | 54.8 | 54.8 | 54.8 | 54.8 |
| Triglyceride | | 5 | 5 | 5 | 5 | 5 | 5 | 5 | 5 | 5 | 5 | 5 | 5 | 5 | 5 | 5 |
| Additive A | | - | - | - | - | - | - | - | - | - | 0.84 | 0.84 | 0.84 | 0.84 | 0.84 | 0.84 |
| Additive B | | - | - | - | - | - | - | - | - | - | - | 3.8 | - | - | - | - |
| Additive C | | - | - | - | - | - | - | - | - | - | - | - | 3.8 | - | - | - |
| Additive D | | - | - | - | - | - | - | - | - | - | - | - | - | 3.8 | 1.9 | - |
| Additive E | | - | - | - | - | - | - | - | - | - | - | - | - | - | 1.9 | 3.8 |
| Percentage to total amount of groups derived from fatty acids in triglyceride [mass%] | C12 | - | - | - | - | - | - | - | - | - | - | - | - | - | - | - |
| | C14 | 1 | 1 | - | - | - | - | - | - | - | 1 | 1 | 1 | 1 | 1 | 1 |
| | C16 | 56 | 42 | 40 | 29 | 3 | 4 | 100 | - | - | 42 | 42 | 42 | 42 | 42 | 42 |
| | C18 | 42 | 57 | 60 | 62 | 37 | 93 | - | 100 | - | 57 | 57 | 57 | 57 | 57 | 57 |
| | C20 | 1 | - | - | - | 9 | 2 | - | - | - | - | - | - | - | - | - |
| | C22 | - | - | - | - | 50 | 1 | - | - | 100 | - | - | - | - | - | - |
| | C24 | - | - | - | - | 1 | - | - | - | - | - | - | - | - | - | - |
| | Unsaturated bond or substituent | No | No | No | No | No | No | No | No | No | No | No | No | No | No | No |
| | Remark | A | B | C | D | E | F | G | H | I | B | B | B | B | B | B |
| Proportion (parts) of triglyceride with respect to 100 parts of olefin-based resin composition | | 5.3 | 5.3 | 5.3 | 5.3 | 5.3 | 5.3 | 5.3 | 5.3 | 5.3 | 12.7 | 14.0 | 14.0 | 14.0 | 14.0 | 14.0 |
| Contact angle [°] | 44.0 mN/m | 119 | 115 | 115 | 121 | 119 | 118 | 109 | 103 | 105 | - | - | - | - | - | - |
| | 35.0 mN/m | 109 | 105 | 97 | 103 | 110 | 90 | 81 | 80 | 72 | 109 | 106 | 103 | 100 | 98 | 68 |

[Table 2]

| | | Comparative Reference Example | | | | | | | |
|---|---|---|---|---|---|---|---|---|---|
| | | 1 | 2 | 3 | 4 | 5 | 6 | 7 | 8 |
| High-melting-point olefin-based resin A | | 100 | 95 | 95 | 95 | 95 | 41.44 | 37.44 | 37.44 |
| Inorganic filler | | - | - | - | - | - | 57.68 | 57.68 | 57.68 |
| Triglyceride | | - | 5 | 5 | 5 | 5 | - | - | - |
| Additive A | | - | - | - | - | - | 0.88 | 0.88 | 0.88 |
| Additive B | | - | - | - | - | - | - | 4 | - |
| Additive C | | - | - | - | - | - | - | - | - |
| Additive D | | - | - | - | - | - | - | - | - |
| Additive E | | - | - | - | - | - | - | - | 4 |
| Percentage to total amount of groups derived from fatty acids in triglyceride [mass%] | C8 | - | - | - | - | 1 | - | - | - |
| | C10 | - | - | - | - | 1 | - | - | - |
| | C12 | - | 100 | - | - | 7 | - | - | - |
| | C14 | - | - | - | 1 | 3 | - | - | - |
| | C16 | - | - | 1 | 21 | 5 | - | - | - |
| | C18 | - | - | 99 | 78 | 64 | - | - | - |
| | C20 | - | - | - | - | 3 | - | - | - |
| | C22 | - | - | - | - | 15 | - | - | - |
| | C24 | - | - | - | - | | - | - | - |
| | Unsaturated bond or substituent | - | No | Yes | Yes | Yes | - | - | - |
| | Remark | | J | K | B:K=1:1 | L | - | - | - |
| Proportion (parts) of triglyceride with respect to 100 parts of olefin-based resin composition | | 0 | 5.3 | 5.3 | 5.3 | 5.3 | 0 | 0 | 0 |
| Contact angle [°] | 44.0 mN/m | 68 | 82 | 83 | Quite uneven; plurality of sites with contact angle of 90° or lower. | 52 | - | - | - |
| | 35.0 mN/m | 48 | 57 | 66 | Quite uneven; plurality of sites with contact angle of 68° or lower. | 37.3 | 46 | 57 | 53 |

The meaning of the alphabets shown in the Remark section is as shown in Table 4.

[Table 3]

| | | Difference in SP value from triglyceride B, E, F |
|---|---|---|
| High-melting-point olefin-based resin A | Linear low-density polyethylene produced with metallocene catalyst; melting point: 116°C; density: 0.924 g/cm$^3$ | - |

(continued)

| | | Difference in SP value from triglyceride B, E, F |
|---|---|---|
| Low-melting-point olefin-based resin B | Ethylene-α-olefin copolymer produced with metallocene catalyst; melting point: 44°C; density: 0.864 g/cm$^3$ | - |
| Filler | Heavy calcium carbonate (average particle size D50: 1.8 μm) | - |
| Additive A | Stearic acid | B |
| Additive B | Olefin oligomer | A |
| Additive C | Low-molecular-weight polyethylene | A |
| Additive D | Ethylene-α-olefin cooligomer | A |
| Additive E | Polyester (polyester consisting of 3.5 mol, 2.5 mol, and 5.5 mol, respectively, of trimethylol propane, adipic acid, and stearic acid; SV: 265; AV: 1.2; OHV; 8.6) | B |
| Additive F | Zinc stearate | B |
| A: Difference in SP value from triglyceride B, E, F: 0.37 or greater<br>B: Difference in SP value from triglyceride B, E, F: less than 0.37 | | |

[Table 4]

| | | |
|---|---|---|
| Triglyceride | A | Hydrogenated palm stearin |
| | B | Hydrogenated palm oil |
| | C | Mixture of palmitic acid triglyceride and stearic acid triglyceride |
| | D | Glyceryl tristearate, technical grade (from Sigma-Aldrich) |
| | E | Hydrogenated high-erucin rapeseed oil |
| | F | Hydrogenated low-erucin rapeseed oil |
| | G | Palmitic acid triglyceride |
| | H | Stearic acid triglyceride |
| | I | Behenic acid triglyceride |
| | J | Lauric acid triglyceride |
| | K | Hydrogenated castor oil |
| | L | Unsaturated triglyceride (80% of C18: unsaturated fatty acid) |
| | M | Hydrogenated coconut oil |

As evident from the results shown in Tables 1 and 2, the films of the Reference Examples have a higher contact angle to the evaluation solutions compared to the films of the Comparative Reference Examples.

{Examples 1 to 8 and Comparative Examples 1 to 5}

(1) Production of Compound:
The components shown in Tables 5 and 6 below were measured in amounts shown in the Tables. The components were mixed with a Henschel mixer (from Kawata MFG Co., Ltd.). The obtained mixture was mixed and kneaded with a biaxial extruder (from Toyo Seiki Seisaku-Sho, Ltd.) at a setting temperature of 180°C and screw rotation speed of 180 rpm, to obtain a pelletized compound. The unit of the compositional makeup shown in Tables 5 and 6 is in "mass%".
(2) Production of Resin Sheet:

Using a 150-mm-wide T-die (from Toyo Seiki Seisaku-Sho, Ltd.), a resin sheet was molded from the compound. As regards the molding conditions, the setting temperature was 150°C and the screw rotation speed was 30 rpm.
(3) Production of Porous Film:
The obtained resin sheet was subjected to uniaxial stretching using a tentering-stretching machine (from IS Giken Co.), to obtain a porous film having a basis weight of 37 g/m$^2$. The stretching temperature and stretching ratio are shown in Tables 5 and 6.

{Evaluation}

The moisture permeability of the porous films obtained in the respective Examples and Comparative Examples was measured according to the following method. The degree of percolation of liquid was also evaluated according to the following method. The results are shown in Tables 5 and 6.

{Moisture Permeability}

The moisture permeability of the porous film was measured according to the following method pursuant to JIS L 1099, Method A-2.

Approximately 25 mL of ion-exchanged water was placed in a glass bottle (LABORAN Screw Tube Bottle No.8 from As One Corporation) having an opening diameter of 2.03 cm (area: 3.23 cm$^2$), and the opening of the glass bottle was covered with a sheet of test piece in a manner that there was no gap therebetween. The test piece was fixed to the glass bottle with a rubber band, and this was employed as an evaluation sample. The mass (W1) of the evaluation sample was measured, and then, the sample was stored in a thermostatic chamber set to 40°C, 20% RH for 10 to 15 hours. After storage, the mass (W2) of the evaluation sample was measured. Also, the storage time (T1; unit: h) was recorded, and the moisture permeability was calculated from the following equation (1).

$$\text{Moisture permeability } (g/(100\ cm^2\cdot h)) = (W1-W2)/(T1\times 3.23)\times 100 \qquad \text{Equation (1):}$$

The value of the moisture permeability of the porous film of the present invention is preferably 0.65 g/(100 cm$^2$·h), more preferably 0.70 g/(100 cm$^2$·h) or greater, further preferably 0.75 g/(100 cm$^2$·h) or greater. In this way, the porous film of the present invention can have high moisture permeability and can release moisture/humidity inside the absorbent article to the outside suitably. On the other hand, the upper limit value of the moisture permeability of the porous film is preferably 4.5 g/(100 cm$^2$·h) or less, more preferably 3.5 g/(100 cm$^2$·h) or less, further preferably 3.0 g/(100 cm$^2$·h) or less, to prevent excessive formation of micropores which may lead to loss of leakage preventability required as a backsheet.

{Degree of Percolation of Liquid}

A piece of porous film cut into a size of at least 50 mm in the machine direction and at least 35 mm in the traverse direction was placed on a sheet of filter paper (from Advantec Toyo Kaisha, Ltd.; No.2; diameter: 70 mm). On this porous film was placed a piece of dry pulp sheet (from Lion Corporation; REED Healthy-Cooking Paper Double (product name); basis weight: 40 g/m$^2$) cut into a size of 25×30 mm.

To the central part of the dry pulp sheet, 0.265 g of wetting tension test solution (surface tension at 25°C: 35 mN/m; from Kanto Chemical Co., Inc.) was injected with a pipette. After injection, a 60-mm-dia., 5-mm-thick cylindrical acrylic plate was placed thereon, and further, a 500 g weight was placed thereon, to conduct pressurization for a maximum of 60 minutes.

The weight was removed after 20 minutes and after 60 minutes, and each time, the degree of percolation of liquid to the filter paper was visually observed, to determine the presence/absence of percolation. This evaluation was conducted for three sets for each example, and the degree of percolation was evaluated according to the following criteria.

A: No percolation was observed on any of the three sheets of filter paper after passage of 60 minutes.
B: No percolation was observed on any of the three sheets of filter paper after passage of 20 minutes.
C: Percolation was observed on at least one sheet after passage of 20 minutes.

[Table 5]

| | | Example | | | | | | | |
|---|---|---|---|---|---|---|---|---|---|
| | | 1 | 2 | 3 | 4 | 5 | 6 | 7 | 8 |
| High-melting-point olefin-based resin A | | 39.40 | 30.44 | 31.40 | 11.46 | 10.82 | 10.82 | 10.82 | 30.44 |
| Low-melting-point olefin-based resin B | | - | - | - | 26.74 | 25.18 | 25.18 | 25.18 | - |
| Density of olefin-based resin composition (g/cm$^3$) | | 0.924 | 0.924 | 0.924 | 0.882 | 0.882 | 0.882 | 0.882 | 0.924 |
| Inorganic filler | | 54.80 | 59.85 | 61.74 | 58.86 | 60.04 | 60.04 | 60.04 | 59.85 |
| Triglyceride | | 5.00 | 5.00 | 2.00 | 2.04 | 1.02 | 1.02 | 1.02 | 5.00 |
| Additive A | | 0.84 | 0.91 | 0.94 | 0.90 | 0.90 | 0.90 | 0.90 | 0.91 |
| Additive B | | - | - | - | - | - | - | - | - |
| Additive C | | - | - | - | - | - | - | - | - |
| Additive D | | - | 3.80 | 3.92 | - | - | - | - | - |
| Additive E | | | - | - | - | - | - | - | 3.80 |
| Additive F | | | | | - | 2.04 | 2.04 | 2.04 | - |
| Percentage to total amount of groups derived from fatty acids in triglyceride [mass%] | C12 | - | - | - | - | - | - | - | - |
| | C14 | 1 | 1 | 1 | 1 | 1 | - | - | 1 |
| | C16 | 42 | 42 | 42 | 42 | 42 | 3 | 4 | 42 |
| | C18 | 57 | 57 | 57 | 57 | 57 | 37 | 93 | 57 |
| | C20 | - | - | - | - | - | 9 | 2 | - |
| | C22 | - | - | - | - | - | 50 | 1 | - |
| | C24 | - | - | - | - | - | 1 | - | - |
| | Unsaturated bond or substituent | No | No | No | No | No | No | No | No |
| | Remark | B | B | B | B | B | E | F | B |
| Proportion (parts) of triglyceride with respect to 100 parts of olefin-based resin composition | | 12.7 | 16.4 | 6.4 | 5.3 | 2.8 | 2.8 | 2.8 | 16.4 |
| Stretching conditions | Ratio | 4.0 | 3.0 | 3.0 | 5.5 | 4.5 | 5.0 | 5.0 | 4.0 |
| | Temperature (°C) | 40 | 40 | 40 | 24 | 40 | 40 | 40 | 40 |
| Degree of percolation | | A | A | A | A | A | A | A | B |
| Moisture permeability (g/100 cm$^2$/h) | | 0.78 | 1.60 | 1.40 | 1.34 | 1.65 | 1.49 | 1.44 | 1.35 |

The meaning of the alphabets shown in the Remark section is as shown in Table 4.

[Table 6]

| | Comparative Example | | | | |
|---|---|---|---|---|---|
| | 1 | 2 | 3 | 4 | 5 |
| High-melting-point olefin-based resin A | 37.44 | 37.44 | 10.77 | 10.77 | 10.77 |
| low-melting-point olefin-based resin B | - | - | 25.23 | 25.23 | 25.23 |
| Density of olefin-based resin composition (g/cm$^3$) | 0.924 | 0.924 | 0.882 | 0.882 | 0.882 |
| Inorganic filler | 57.68 | 57.68 | 60.04 | 60.04 | 60.04 |

(continued)

| | | Comparative Example | | | | |
|---|---|---|---|---|---|---|
| | | 1 | 2 | 3 | 4 | 5 |
| Triglyceride | | - | - | 1.02 | 1.02 | 1.02 |
| Additive A | | 0.88 | 0.88 | 0.9 | 0.9 | 0.9 |
| Additive D | | 4.00 | - | - | - | - |
| Additive E | | - | 4.00 | - | - | - |
| Additive F | | - | - | 2.04 | 2.04 | 2.04 |
| Percentage to total amount of groups derived from fatty acids in triglyceride [mass%] | C8 | - | - | - | - | 6 |
| | C10 | - | - | - | - | 5 |
| | C12 | - | - | - | - | 48 |
| | C14 | - | - | - | 1 | 19 |
| | C16 | - | - | 1 | 21 | 10 |
| | C18 | - | - | 99 | 78 | 12 |
| | C20 | - | - | - | - | - |
| | C22 | - | - | - | - | - |
| | C24 | - | - | - | - | - |
| | Unsaturated bond or substituent | - | - | Yes | Yes | No |
| | Remark | - | - | K | B:K=1:1 | M |
| Proportion (parts) of triglyceride with respect to 100 parts of olefin-based resin composition | | 0 | 0 | 2.8 | 2.8 | 2.8 |
| Stretching conditions | Ratio | 4.5 | 4.0 | 4.5 | 5.0 | 4.5 |
| | Temperature (°C) | 40 | 40 | 40 | 40 | 40 |
| Degree of percolation | | C | C | C | C | C |
| Moisture permeability (g/100 $cm^2$/h) | | 0.81 | 0.97 | 1.53 | 1.74 | 1.75 |

The meaning of the alphabets shown in the Remark section is as shown in Table 4.

As evident from the results shown in Tables 5 and 6, the porous films obtained in the respective Examples have excellent liquid percolation preventability while maintaining excellent moisture permeability.

{Comparative Example 6}

A porous film was produced in the same manner as in Example 1 except that the components shown in Table 7 were used, and the following evaluation was conducted. Note that Table 7 also shows Example 4.

{Evaluation}

Two sheets of the porous films were placed on top of one another and were pressed using a Labo Press (from Toyo Seiki Seisaku-Sho, Ltd.) at 50°C, 127 MPa for 1 minute. Next, the sheets were cooled at ordinary temperature for 5 minutes, and the pair of sheets was employed as a sample for blocking evaluation. Blocking evaluation was conducted by peeling the two films of the prepared evaluation sample apart by hand, to see whether or not blocking occurred. This evaluation was conducted for three sets for each example, and blocking was evaluated according to the following criteria.

A: When peeled by hand, all three pairs peeled easily.
B: When peeled by hand, at least one pair did not peel easily and the film got torn.

[Table 7]

| | | Example | Comparative Example |
|---|---|---|---|
| | | 4 | 6 |
| High-melting-point olefin-based resin A | | 11.46 | 11.12 |
| Low-melting-point olefin-based resin B | | 26.74 | 25.95 |
| Density of olefin-based resin composition ($g/cm^3$) | | 0.882 | 0.882 |
| Inorganic filler | | 58.86 | 61.99 |
| Triglyceride | | 2.04 | - |
| Additive A | | 0.90 | 0.95 |
| Additive B | | - | - |
| Additive C | | - | - |
| Additive D | | - | - |
| Percentage to total amount of groups derived from fatty acids in triglyceride [mass%] | C12 | - | - |
| | C14 | 1 | - |
| | C16 | 42 | - |
| | C18 | 57 | - |
| | C20 | - | - |
| | C22 | - | - |
| | C24 | - | - |
| | Unsaturated bond or substituent | No | - |
| | Remark | B | - |
| Proportion (parts) of triglyceride with respect to 100 parts of olefin-based resin composition | | 5.3 | 0 |
| Blocking | | A | B |

The meaning of the alphabet shown in the Remark section is as shown in Table 4.

As evident from the results shown in Table 7, the porous film obtained in Example 4 was less prone to blocking than Comparative Example 6.

Industrial Applicability

As described in detail above, the present invention can provide a porous film having better leakage preventability than ever before while maintaining satisfactory moisture permeability.

## Claims

1. A porous film comprising at least an olefin-based resin composition, an inorganic filler, and a triglyceride, wherein:

   a content of the filler is from 50 to 400 parts by mass with respect to 100 parts by mass of the olefin-based resin composition;
   a content of the triglyceride is from 0.1 to 30 parts by mass with respect to 100 parts by mass of the olefin-based resin composition; and
   the triglyceride includes a group derived from a fatty acid having from 16 to 22 carbon atoms, the group being a hydrocarbon group having neither an unsaturated bond nor a substituent.

2. The porous film according to claim 1, wherein the triglyceride is

(A) a mixture of a triglyceride including, in a single molecule, at least a group derived from a fatty acid having 18 carbon atoms and a triglyceride including, in a single molecule, at least a group derived from a fatty acid having from 16 to 22 carbon atoms (except for a fatty acid having 18 carbon atoms), or
(B) a triglyceride including, in a single molecule, at least one group derived from a saturated fatty acid having 18 carbon atoms and at least one group derived from a saturated fatty acid having from 16 to 22 carbon atoms (except for a fatty acid having 18 carbon atoms).

**3.** The porous film according to claim 1 or 2, wherein the triglyceride is a triglyceride including, in a single molecule, at least one group derived from a saturated fatty acid having 18 carbon atoms and at least one group derived from a saturated fatty acid having from 16 to 22 carbon atoms (except for a fatty acid having 18 carbon atoms).

**4.** The porous film according to any one of claims 1 to 3, wherein, with respect to a total amount of groups derived from fatty acids included in all the triglyceride, from 28 to 96 mass% is a group derived from a fatty acid having 18 carbon atoms.

**5.** The porous film according to any one of claims 1 to 4, wherein, with respect to the total amount of groups derived from fatty acids included in all the triglyceride, from 28 to 70 mass% is a group derived from a fatty acid having 18 carbon atoms.

**6.** The porous film according to any one of claims 1 to 5, wherein, with respect to the total amount of groups derived from fatty acids included in all the triglyceride, from 28 to 68 mass% is a group derived from a fatty acid having 18 carbon atoms and from 26 to 70 mass% is a group derived from a fatty acid having 16 carbon atoms.

**7.** The porous film according to any one of claims 1 to 5, wherein, with respect to the total amount of groups derived from fatty acids included in all the triglyceride, from 28 to 47 mass% is a group derived from a fatty acid having 18 carbon atoms and from 40 to 60 mass% is a group derived from a fatty acid having 22 carbon atoms.

**8.** The porous film according to any one of claims 1 to 7, wherein the groups derived from fatty acids included in all the triglyceride are hydrocarbon groups not having an unsaturated bond.

**9.** The porous film according to any one of claims 1 to 8, wherein the groups derived from fatty acids included in all the triglyceride are hydrocarbon groups not having a substituent.

**10.** The porous film according to any one of claims 1 to 9, wherein:

the olefin-based resin composition has a density of less than 0.900 g/$cm^3$; and
the olefin-based resin composition has a density of 0.840 g/$cm^3$ or greater.

**11.** The porous film according to any one of claims 1 to 10, wherein:

the olefin-based resin composition includes a low-melting-point olefin-based resin having a melting point below 90°C;
the low-melting-point olefin-based resin has a density of 0.895 g/$cm^3$ or less; and
the low-melting-point olefin-based resin has a density of 0.840 g/$cm^3$ or greater.

**12.** The porous film according to claim 11, comprising from 30 to 95 parts by mass of the low-melting-point olefin-based resin in 100 parts by mass of the olefin-based resin composition.

**13.** The porous film according to any one of claims 1 to 12, wherein:

the olefin-based resin composition includes a high-melting-point olefin-based resin having a melting point of 95°C or higher; and
the high-melting-point olefin-based resin has a density of from 0.900 to 0.950 g/$cm^3$.

**14.** The porous film according to claim 13, wherein the high-melting-point olefin-based resin includes polyethylene.

**15.** The porous film according to claim 14, wherein the high-melting-point olefin-based resin includes a linear low-density polyethylene.

**16.** The porous film according to claim 15, wherein the linear low-density polyethylene includes a linear low-density polyethylene polymerized with a metallocene catalyst.

**17.** The porous film according to claim 11 or 12, wherein the low-melting-point olefin-based resin includes an ethylene-α-olefin copolymer.

**18.** The porous film according to claim 17, wherein the ethylene-α-olefin copolymer includes an ethylene-α-olefin copolymer polymerized with a metallocene catalyst.

**19.** The porous film according to any one of claims 1 to 10, wherein:

the olefin-based resin composition includes a low-melting-point olefin-based resin having a melting point below 90°C and a high-melting-point olefin-based resin having a melting point of 95°C or higher;
the high-melting-point olefin-based resin includes a linear low-density polyethylene polymerized with a metallocene catalyst; and
the low-melting-point olefin-based resin includes an ethylene-α-olefin copolymer polymerized with a metallocene catalyst.

**20.** The porous film according to any one of claims 1 to 19, further comprising an additive, wherein:

the additive includes at least a first additive whose difference in SP value from the triglyceride is less than 0.37; and
a content of the first additive is from 0.01 to 3.5 parts by mass with respect to 100 parts by mass in total of the olefin-based resin composition, the inorganic filler, and the triglyceride.

**21.** The porous film according to any one of claims 1 to 20, further comprising an additive, wherein:

the additive includes at least a second additive whose difference in SP value from the triglyceride is 0.37 or greater; and
a content of the second additive is from 0.01 to 10 parts by mass with respect to 100 parts by mass in total of the olefin-based resin composition, the inorganic filler, and the triglyceride.

**22.** The porous film according to claim 21, wherein the difference in SP value of the second additive from the triglyceride is from 0.37 to 4.00.

**23.** The porous film according to any one of claims 1 to 22, wherein the inorganic filler has an average particle size of 30 μm or less.

**24.** The porous film according to any one of claims 1 to 22, wherein calcium carbonate is used as the inorganic filler.

**25.** The porous film according to any one of claims 1 to 24, wherein a content of the inorganic filler is from 60 to 300 parts by mass with respect to 100 parts by mass of the olefin-based resin composition.

**26.** The porous film according to any one of claims 1 to 25, wherein an amount of the triglyceride to be blended is from 1.0 to 20 parts by mass with respect to 100 parts by mass of the olefin-based resin composition.

**27.** The porous film according to any one of claims 1 to 26, wherein the porous film is for use in an absorbent article.

**28.** The porous film according to claim 27, wherein the porous film is for use as a leak-proof sheet in the absorbent article.

**29.** The porous film according to claim 27, wherein the porous film is for use as a leak-proof cuff in the absorbent article.

**30.** An absorbent article comprising the porous film according to any one of claims 1 to 29.

**31.** The absorbent article according to claim 30, comprising:

a topsheet forming a skin-facing surface;
a leak-proof sheet forming a non-skin-facing surface; and

a liquid-retentive absorbent member arranged between the topsheet and the leak-proof sheet, wherein:
the leak-proof sheet is the porous film.

**32.** The absorbent article according to claim 30, comprising:

a topsheet forming a skin-facing surface;
a leak-proof sheet forming a non-skin-facing surface; and
a liquid-retentive absorbent member arranged between the topsheet and the leak-proof sheet, wherein:

leak-proof cuffs are provided respectively on the skin-facing surface's both lateral sides along a longitudinal direction; and
the leak-proof cuff is the porous film.

**INTERNATIONAL SEARCH REPORT**

International application No. **PCT/JP2022/016907**

**A. CLASSIFICATION OF SUBJECT MATTER**

***C08J 9/00***(2006.01)i
FI: C08J9/00 A CES

According to International Patent Classification (IPC) or to both national classification and IPC

**B. FIELDS SEARCHED**

Minimum documentation searched (classification system followed by classification symbols)

C08J9/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 62-10141 A (KAO CORP.) 19 January 1987 (1987-01-19) claims, p. 2, lower left column, line 1 to lower right column, the last line, p. 3, lower left column, lines 13-18, examples | 1, 8-9, 13-16, 20, 23-28, 30 |
| Y | | 10-19, 28-32 |
| A | | 2-7, 21-22 |
| X | JP 2001-302828 A (MITSUBISHI PLASTICS INC.) 31 October 2001 (2001-10-31) claims, paragraphs [0011]-[0015], [0021], examples | 1, 8-9, 13-16, 23-28, 30 |
| Y | | 10-19, 28-32 |
| A | | 2-7, 21-22 |
| Y | JP 2020-131694 A (MITSUBISHI CHEMICAL CORP.) 31 August 2020 (2020-08-31) claims, paragraphs [0017]-[0033], [0037]-[0047], examples | 10-19, 28-32 |
| A | | 2-7, 21-22 |
| Y | JP 2020-130951 A (KAO CORP.) 31 August 2020 (2020-08-31) claims, paragraph [0024], examples | 28-32 |
| A | | 2-7, 21-22 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 June 2022** | **28 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/016907**

| Patent document cited in search report | Publication date (day/month/year) | Patent family member(s) | Publication date (day/month/year) |
|---|---|---|---|
| JP 62-10141 A | 19 January 1987 | (Family: none) | |
| JP 2001-302828 A | 31 October 2001 | (Family: none) | |
| JP 2020-131694 A | 31 August 2020 | (Family: none) | |
| JP 2020-130951 A | 31 August 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S6210141 A
- JP 2001302828 A
- JP 2016089009 A